(19) Europäisches Patentamt
European Patent Office
Office européen des brevets

(11) **EP 2 165 726 A2**

(12) **EUROPÄISCHE PATENTANMELDUNG**

(43) Veröffentlichungstag:
**24.03.2010 Patentblatt 2010/12**

(51) Int Cl.:
***A61M 16/00*** (2006.01)    *A61B 5/091* (2006.01)

(21) Anmeldenummer: **09075437.5**

(22) Anmeldetag: **21.09.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(30) Priorität: **22.09.2008   DE 102008048824**

(71) Anmelder: **Borm, Hans-Jürgen**
**12524 Berlin (DE)**

(72) Erfinder: **Borm, Hans-Jürgen**
**12524 Berlin (DE)**

(54) **Verfahren und Einrichtung zur automatischen Ermittlung eines Beatmungsvolumens bei der maschinellen Beatmung, unter Berücksichtigung von Leckagen**

(57)    Bezeichnung: Verfahren und Einrichtung zur automatischen Ermittlung eines Beatmungsvolumens bei der maschinellen Beatmung, unter Berücksichtigung von Leckagen.

Zusammenfassung: Die Erfindung betrifft ein Verfahren zur automatischen Ermittlung eines Beatmungsvolumens bei der maschinellen Beatmung, unter den Bedingungen wechselnder parasitärer Leckagen und Nominalleckagen sowie einer Einrichtung zur künstlichen Beatmung mit Volumensicherung. Erfindungsgemäß ist vorgesehen, unter Verwendung des ermittelten realen Titalvolumens und einer Steuereinheit, Beatmungsgeräte automatisch so zu beeinflussen, dass eine permanente Korrektur eines abzugebenden Titalvolumens und vorgegebener Inspirationsdruck-Begrenzungen erfolgt.

Figur 6    Voraussage Vt P mit Hilfe der Leckageöffnung

EP 2 165 726 A2

**Beschreibung**

**Einleitung**

Die Erfindung betrifft ein Verfahren zur Ermittlung des Beatmungsvolumens von künstlich beatmeten Lungen, unter den Bedingungen wechselnder parasitärer Leckagen und unterschiedlicher Nominalleckagen, wobei mit Hilfe der vorgeschlagenen Berechnungen, das echte inspiratorische und/oder exspiratorische Leckagevolumen ermittelt wird und daraus resultierend, bezugnehmend auf die gemessenen inspiratorischen und/oder exspiratorischen Volumina, das Titalvolumen (im folgenden reales Titalvolumen genannt) errechnet wird, welches tatsächlich am Gasaustausch in der Patientenlunge teilgenommen hat.

Die Erfindung betrifft darüber hinaus eine Einrichtung in Form einer Steuereinheit für Beatmungsgeräte, welche das im vorgeschlagenen Verfahren ermittelte reale Titalvolumen benutzt, um die Zufuhr von Inspirationsvolumina und Druckbegrenzungen zu steuern und zu korrigieren, sowie Messungen der dynamischen Atemmechanik genauer durchführen zu können.

**Definitionen**

Die in den folgenden Erläuterungen genannten Fachbegriffe haben in Rahmen der vorliegenden Anmeldung und innerhalb der beschriebenen maschinellen Beatmung folgende Bedeutung und Funktionen:

Inspiratorisches Titalvolumen (Beatmungsvolumen)

Bei der künstlichen Beatmung wird der Austausch der verbrauchten Atemluft mittels Beatmungsgerät unterstützt. Die Menge Luft, die pro Atemzug in die Lunge verbracht wird, ist das inspiratorische Titalvolumen (im folgenden "$V_{ti}$" genannt).

Exspiratorisches Titalvolumen.

Die Menge Luft, die jeweils wieder ausgeatmet wird, bezeichnet man als exspiratorisches Titalvolumen (im Folgenden "$V_{te}$" genannt).

Reales Titalvolumen

Das Beatmungsvolumen, welches bei jedem Atemzug tatsächlich in der Lunge am Gasaustausch teilgenommen hat und wieder ausgeatmet wird (im Folgenden "$V_t P$" genannt).

Inspirationsdruck

Der Druck, der bei der maschinellen Beatmung bei der Einatmung in der Lunge und dem Beatmungssystem erreicht wird (im Folgenden "IPAP" genannt).
Inspirationsdruckbegrenzung Vom Beatmungsgerät max. zugelassener Druck während der Inspiration, um der Lunge ein vorgegebenes Beatmungsvolumen zuzuführen (im Folgenden "$P_{max}$" genannt).

Exspirationsdruck

Der Druck, der bei der maschinellen Beatmung am Ende der Ausatmung in der Lunge und dem Beatmungssystem bis zur nächsten Einatmung gehalten werden soll (im Folgenden "PEEP" genannt).

Inspirationszeit

Die Zeit, in der das Beatmungsvolumen in die Lunge verabreicht wird (im Folgenden "$T_i$" genannt.

Exspirationszeit

Im allgemeinen wird damit die gesamte Zeit von Beginn der Ausatmung bis zum Beginn der nächsten Einatmung definiert. Da eine evtl. Leckage in der Atempause jedoch für den zeitlichen Verlauf der Leckagevolumina keine Rolle spielt, wird hiermit nur die dafür relevante Zeit von Beginn bis zum Ende der Ausatmung bezeichnet (im Folgenden "$T_e$" genannt.

Leckageöffnung

Die Öffnung innerhalb des Beatmungssystems, aus der ständig oder zeitweise ein Teil der Luft, die einer Lunge bei der maschinellen Beatmung zugeführt wird oder die von dieser ausgeatmet wird, in die Umgebung entweicht (im Folgenden "LÖ" genannt).

Nominalleckage

Die Öffnung innerhalb des Beatmungssystems mit einer definierten Größe, aus der ständig ein Teil der Luft, die einer Lunge bei der maschinellen Beatmung zugeführt werden soll oder die von dieser ausgeatmet wird, in die Umgebung entweicht. Diese Nominalleckage wird bewußt in sogenannte offene Beatmungssysteme eingesetzt, um auch ohne gesteuertes Ausatemventil eine aktive Ausatmung der Lunge zu ermöglichen und das $CO_2$ aus der Ausatemluft der Lunge auszuspülen. Dies wird durch ein passives Exspirationsventil (s. g. Whisper Swivel) mit einer oder mehrerer Öffnungen oder einer Maske mit entsprechenden Öffnungen realisiert. Dabei sind je nach Hersteller und Typ Unterschiede im Leckageflow von 10% bis zu 30% bekannt.

Parasitäre Leckage

Die Öffnung innerhalb des Beatmungssystems unbekannter Größe, aus der ständig oder zeitweise ein Teil der Luft, die einer Lunge bei der maschinellen Beatmung zugeführt werden soll oder die von dieser ausgeatmet wird, in die Umgebung entweicht. Bei der nichtinvasiven Beatmung (auch Maskenbeatmung genannt) treten mehr oder weniger häufig wechselnde parasitäre Leckagen an den Kontaktstellen zw. dem verwendeten Maskensystem und dem Gesicht des beatmeten Patienten auf. Bei der invasiven Beatmung (über Trachiostoma) werden teilweise bewußt parasitäre Leckagen in Kauf genommen (z. B. bei der Verwendung spezieller Beatmungskanülen, ungeblockte Kanüle bei der Beatmung von Kindern, teilweise ungeblockte Kanülen um den Patienten das Sprechen zu ermöglichen usw.). Diese Leckagen sind nicht konstant und unterliegen ständiger Veränderung.

Inspiratorisches Leckagevolumen

Die Menge an Beatmungsvolumen, welche vom Beatmungsgerät in Richtung Lunge abgegeben wird jedoch auf dem Weg dorthin in die Umgebungsluft entweicht.

Exspiratorisches Leckagevolumen

Die Menge an Ausatemvolumen, welche von der Lunge in Richtung Beatmungsgerät abgegeben wird, jedoch auf dem Weg dorthin in die Umgebungsluft entweicht.

Druckgesteuerten Beatmung

Beatmungsform, bei der vom Beatmungsgerät solange ein Luftstrom zur Lunge geleitet wird, bis ein festgelegter Inspirationsdruck erreicht ist, dann entsprechend einer vorgegebenen Zeit gehalten wird, bis die Ausatmung eingeleitet wird.

Druckgesteuerte Beatmung mit Zielvolumen

Druckgesteuerte Beatmung, bei der das Beatmungsgerät das erreichte Inspiratorische Titalvolumen überwacht und bei Unterschreitung dessen, den Inspirationsdruck innerhalb festgelegter Grenzen erhöht, sowie bei Überschreitung wieder absenkt.

Volumengesteuerte Beatmung

Beatmungsform, bei der vom Beatmungsgerät ein festgelegtes Beatmungsvolumen zur Lunge geleitet wird.

Volumengesteuerte Beatmung mit Druckregulation

Volumengesteuerte Beatmung, bei der das Beatmungsgerät innerhalb festgelegter Grenzen einen höheren oder niedrigeren Grenzdruck zuläßt, je nachdem, ob das festgelegte Beatmungsvolumen verabreicht werden konnte oder nicht.

Atemmechanik

Der Strömungswiderstand (Resistance), den die Atemwege dem Luftstrom entgegensetzen und die Dehnfähigkeit (Compliance) der Lunge.

Druckkurve

Der zeitlicher Verlauf des Drucks im Beatmungssystem.

Flowkurve

Der zeitlicher Verlauf des Luftstroms (Flow) im Beatmungssystem.

Beatmungszyklus

Zu einem kpl. Beatmungszyklus gehört die Inspiration, Exspiration und die Atempause bis zum Beginn der nächsten Inspiration.

**Stand der Technik**

Die am Markt befindlichen Beatmungsgeräte verfügen i.d.R. über eine Berechnung und Anzeige des inspiratorischen Titalvolumens (im folgenden $V_{ti}$ genannt). Dieses wird mittels geräte- oder patientennah angebrachter Sensorik gemessen. Evtl. vorhandene parasitäre Leckagen bleiben dabei unberücksichtigt.

Bei Beatmungsgeräten, die zur Beatmung mit Doppelschlauchsystemen vorgesehen sind, wird zusätzlich das exspiratorische Titalvolumen (im folgenden $V_{te}$ genannt) gemessen und angezeigt. Auch hier ohne Berücksichtigung evtl. vorhandener parasitärer Leckagen.

Das (reale) Titalvolumen ($V_t$ P), welches aktiv am Gasaustausch in der Patientenlunge teilnimmt, stimmt unter den Bedingungen vorhandener Leckage, mit dem vom Beatmungsgerät üblicherweise ermittelten $V_{ti}$ und/oder $V_{te}$ nicht annähernd überein.

Es gibt bereits Beatmungsgeräte, die Nominalleckagen berücksichtigen. Diese haben eine Auswahlmöglichkeit des Maskentyps mit entsprechender integrierter Nominalleckage oder separater Nominalleckage. Das dient zur Präzisierung des Alarmmanagements. Die Auswahl der Nominalleckagen ist jedoch jeweils eingeschränkt und umfaßt nicht alle am Markt befindlichen Versionen.

Vereinzelt sind in den Beatmungsgeräten Leckageberechnungen integriert, die lediglich eine prozentuale Differenz zwischen dem gemessenen inspiratorischen und exspiratorischen Volumen darstellen. Diese können jedoch weder in die Anzeigen der Titalvolumina noch in die Steuerung und Regulation des Beatmungsgerätes bzgl. einer Volumen- oder Druckregulation einfließen, da die Anteile der inspiratorischen und exspiratorischen Leckage nicht ermittelt werden.

Einzelne Steuerungen von Beatmungsgeräten berücksichtigen bei der Regulation des zulässigen Inspirationsdrucks oder der Abgabe eines Beatmungsvolumens die sich verändernde Resistance und/oder Compliance der Lunge.

Bei auftretenden parasitären Leckagen gibt es in Abhängigkeit von deren Größe z.T. erhebliche Abweichungen von einer zuvor optimierten Beatmung. Diese Abweichungen können zu einem erheblichen Mehraufwand in der Therapieverlaufskontrolle führen, da die Abweichungen der tatsächlichen Beatmung von der medizinisch vorgegebenen nur durch zusätzliche Untersuchungen erkannt werden können. Erst im nachhinein dessen, können bisher dementsprechend manuelle Korrekturen bei den Beatmungsparametern zur Anpassung der künstlichen Beatmung bzw. der Unterstützung der Spontanatmung vorgenommen werden.

Treten bei den bekannten druckgesteuerten Beatmungen zeitgleich mit den Veränderungen der Resistance und/oder Compliance der Patientenlunge, parasitäre Leckagen im Beatmungssystem auf, werden diese Veränderungen der Resistance und/oder Compliance bei einer automatischen Korrektur eines Zielvolumens oder einer Druckbegrenzung $P_{max}$ nicht oder falsch berücksichtigt, da die Druck/Flowkurven und die gemessenen Volumina durch die Leckagen verfälscht werden.

Bei den bekannten volumengesteuerten Beatmungen werden parasitäre Leckagen nicht ausgeglichen, da die Leckagen nicht ermittelt und somit nicht zur Korrektur der Steuerung verwendet werden können.

Treten bei Volumenmodi mit Druckregulation eines $P_{max}$ zeitgleich mit den Veränderungen der Resistance und/oder Compliance der Lunge parasitäre Leckagen im Beatmungssystem auf, können die Beatmungsgeräte diese Veränderungen nicht mehr qualifizieren, da die gemessenen Druck/Flowkurven und die gemessenen Volumina durch die parasitären Leckagen verfälscht werden. Das gemessene $V_{ti}/V_{te}$ stimmt mit dem tatsächlichen Volumen, welches am Gasaustausch teilnimmt, nicht überein, was zwangsläufig zu einer Fehlbeurteilung eines zu regulierenden Inspirationsvolumens und/oder Inspirationsdrucks führt. Aus diesen Gründen sind bisher jegliche Volumenbeatmungen nur unzureichend für die nichtinvasive Beatmung geeignet.

Beatmungsgeräte, die über eine integrierte Messung der dynamischen Atemmechanik (Messung der Resistance und Compliance während der Beatmung ohne Beeinflussung der Inspiration und Exspiration) verfügen, können diese Manöver nur in geschlossenen Beatmungssystemen durchführen, da die Nominalleckagen bei Beatmung mit offenen Beatmungssystemen die notwendige Analyse der Druck/Flowkurven verfälschen.

## Aufgabenstellung

Es ist Aufgabe der vorliegenden Erfindung, ein Verfahren zur automatischen Ermittlung und Darstellung des realen Titalvolumens ($V_t$ P), welches bei jedem Atemzug am Gasaustausch in der Lunge teilnimmt, zu schaffen.

Weiterhin soll diese $V_t$ P zur automatischen Beeinflussung der Steuerung der Beatmung genutzt werden. Die Einrichtung zur automatischen Steuerung einzelner Beatmungsparameter unter Berücksichtigung des neuen $V_t$ P soll zu einer stabileren Versorgung der Lunge mit frischem Atemgas führen.

Um allen Bedingungen in der Praxis gerecht zu werden, ist es notwendig, unterschiedliche Verfahren zur Ermittlung des $V_t$ P in die Steuerung eines Beatmungsgerätes zu integrieren. Zur Realisierung dieser Verfahren werden verschiedene Parameter berechnet, die bisher bei den bekannten Verfahren zur Steuerung von maschinellen Beatmungen nicht berechnet wurden. Unter anderem wird unter Anwendung der Erfindung, die vorhandene Leckageöffnung sowie die tatsächlichen Anteile der inspiratorischen und exspiratorischen Leckage ermittelt.

Eine weitere Aufgabe der Erfindung ist es, zwischen Nominalleckagen und parasitärer Leckage zu unterscheiden. Da sich die Nominalleckagen je nach Hersteller unterscheiden, wird die tatsächliche Nominalleckage durch Messungen während einer Kalibration ermittelt. Die ermittelte LÖ wird bei Beatmung mit offenen Systemen in die Anteile der Nominalleckage (wurde zuvor durch die Kalibrierung ermittelt) und der parasitären Leckage aufgeteilt.

Unter Ausnutzung der Kalibration der Nominalleckage soll die Messung der dynamischen Atemmechanik einer Lunge auch im offenen Beatmungssystem durchführbar sein.

## Ausführungsbeispiele

Die Erfindung bzgl. der Verfahren wird im folgenden anhand jeweils eines Ausführungsbeispiels in den Zeichnungen beschrieben.

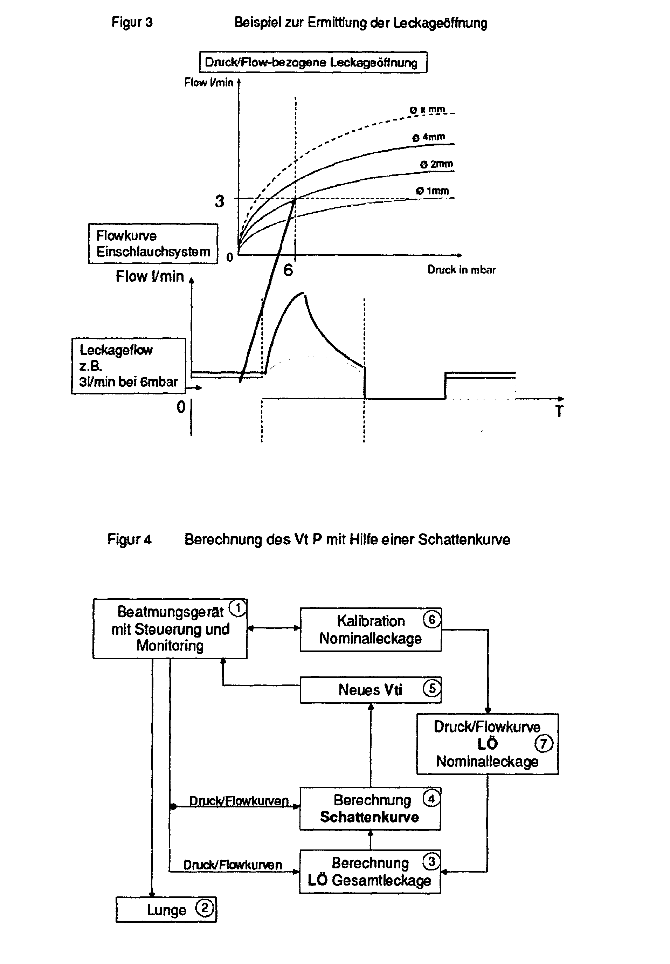

Die Figuren 3 und 8 bis 14 dienen weiteren Erläuterungen und werden nicht separat erklärt. Die Figuren 3, 5, 12 und 13 sind zum besseren Verständnis der Erfindung grafische Darstellungen, werden jedoch in den Programmteilen der Steuerung mittels numerischer Berechnungsformeln genutzt.

Figur 1, 2 und 4

Die prinzipiellen Funktionen eines herkömmlichen Beatmungsgerätes (1) bestehen darin, dass die Steuerung und Überwachung aller Funktionen CPU-gestützt erfolgt. In einem Speicherbereich wird das Steuerprogramm zur Verfügung gestellt und dort werden Daten, welche während der Beatmung gesammelt werden, zur weiteren Verwendung abgelegt. Weiterhin verfügt ein Beatmungsgerät über ein Monitoring, auf dem vorgegebene und gemessene Beatmungsparameter numerisch und/oder graphisch dargestellt werden. Als Bedieneinheit zur Eingabe von Sollwerten oder dem Abrufen von Daten während der maschinellen Beatmung dienen Tastaturen, Drehknöpfe oder Touchsceenbildschirme. Die zur Steuerung notwendigen Istwerte wie z. B. Druck und Flow, werden mittels elektrischer Sensoren geräteintern oder patientennah gemessen. Mittels Steuerung einer Turbine oder von Druckgas-ventilen, wird der zu beatmenden Lunge ein definiertes Beatmungsvolumen oder ein Beatmungsdruck über eine definierte Zeit mit einer definierten Beatmungsfrequenz zugeführt. In aller Regel verfügt ein modernes Beatmungsgerät über eine Erkennung (Triggereinrichtung), die dem Gerät signalisiert, dass der Patient zusätzliche Atemzüge benötigt, welche dann vom Gerät unterstützt werden.

Figur 1, 2, 4 und 7

Die Beschreibung der Kalibration der Nominalleckage (Fig. 1+2 Programmteil 7; Fig. 4 Programmteil 6; Fig. 7 Programmteil 3) erfolgt in der "Beschreibung der Einrichtung", da eine sinnvolle Erläuterung nur im Kontext zwischen dem Programmteil und der Einrichtung erfolgen kann.

Figur 1

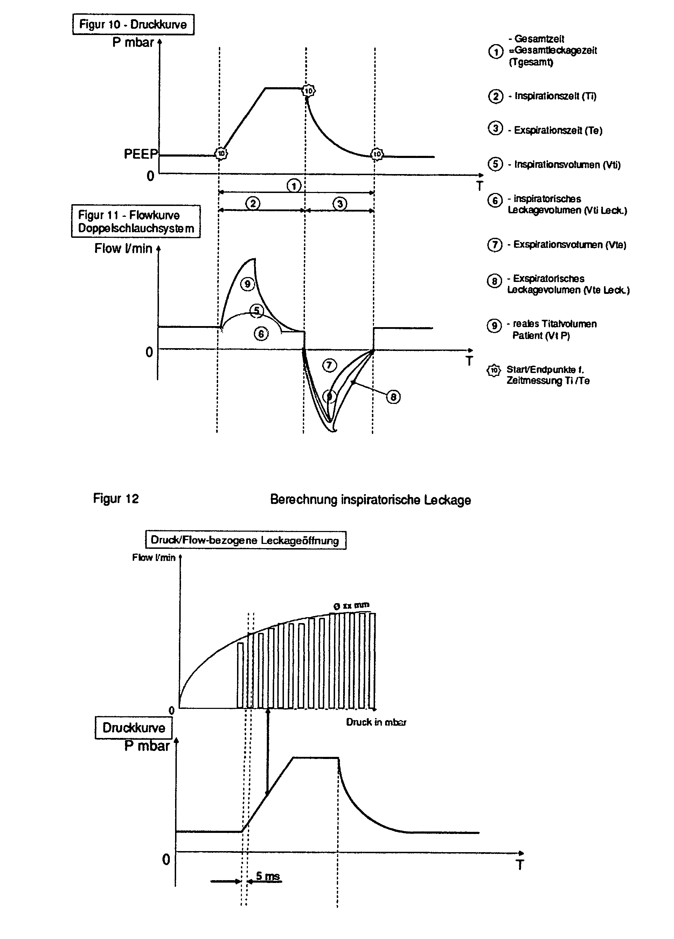

Die genannte Einrichtung (Beatmungsgerät) ist in einer bevorzugten Variante zusätzlich zur Messung des Inspirationsvolumens mit einer integrierten Messung des Exspirationsvolumens ausgestattet.
Im Programmteil 3 werden zusätzlich aus den üblichen Druck- Flowkurven, die Werte $T_i$ (Fig. 10 (2)) und $T_e$ (Fig. 10 (3)) automatisch gemessen. Die in Fig. 10 eingezeichneten Start/Stoppunkte (10) werden dabei zur Messung genutzt. Aus den während der Zeit $T_i$ gemessenen Drücken wird der Mittelwert Pi mittel errechnet.
Im Programmteil 4 wird zunächst durch Differenzbildung zw. $V_{ti}$ und $V_{te}$ die Gesamtleckage Vt Leck berechnet. Danach werden die anteiligen Leckagen berechnet. Da sich die Leckagen ($V_{ti}$ Leck und $V_{te}$ Leck) statisch betrachtet proportional zu den Zeiten ($T_i$ und $T_e$) verhalten, wird mit der folgenden Berechnung das inspiratorische Leckagevolumen errechnet:

$$\text{Vti Leck} = \frac{\text{Vt Leck}}{\text{Ti} + \text{Te}} \times \text{Ti}$$

Als zweite Variante kann das exspiratorische Leckagevolumen errechnet werden.

$$\text{Vte Leck} = \frac{\text{Vt Leck}}{\text{Ti} + \text{Te}} \times \text{Te}$$

Im nächsten Schritt wird im Programmteil 5 automatisch mittels Differenzbildung zw. $V_{ti}$ und Vti Leck das $V_t$ P errechnet. Auch mittels Summenbildung von $V_{te}$ und $V_{te}$ Leck kann das $V_t$ P hier errechnet werden. Im Programmteil 6 wird durch automatischen Vergleich zw. dem Sollwert in Form eines Beatmungsvolumens (Vt) oder eines Zielvolumens (Ziel $V_t$) und dem ermittelten $V_t$ P ein Korrekturwert gebildet. Dieser beeinflußt dann automatisch die Steuerung für den nächsten Atemhub.

Da bei Beatmungen mit Nominalleckage eine Differenzierung zwischen einer Nominalleckage und parasitärer Leckagen notwendig ist, wird mittels Kalibrationsprogramm 7 die Nominalleckage ermittelt und die entsprechende DruckFlowkurve im Programmteil 8 zur weiteren Verwendung gespeichert. Diese DruckFlowkurve wird programmgemäß parallel zur inspiratorischen Druck- Flowkurve alle 5 ms abgetastet und durch Integration über die Inspirationszeit, das inspiratorische Leckagevolumen, welches durch die Nominalleckage verursacht wurde, errechnet (siehe Fig. 12). Durch automatische Differenzierung zw. der zuvor ermittelten $V_{ti}$ Leck, welches dem inspiratorischen Gesamtleckagevolumen entspricht und dem nunmehr bekannten Leckagevolumen, welches durch die Nominalleckage verursacht wurde, können die unterschiedlichen Anteile im Monitoring angezeigt werden.

Figur 2

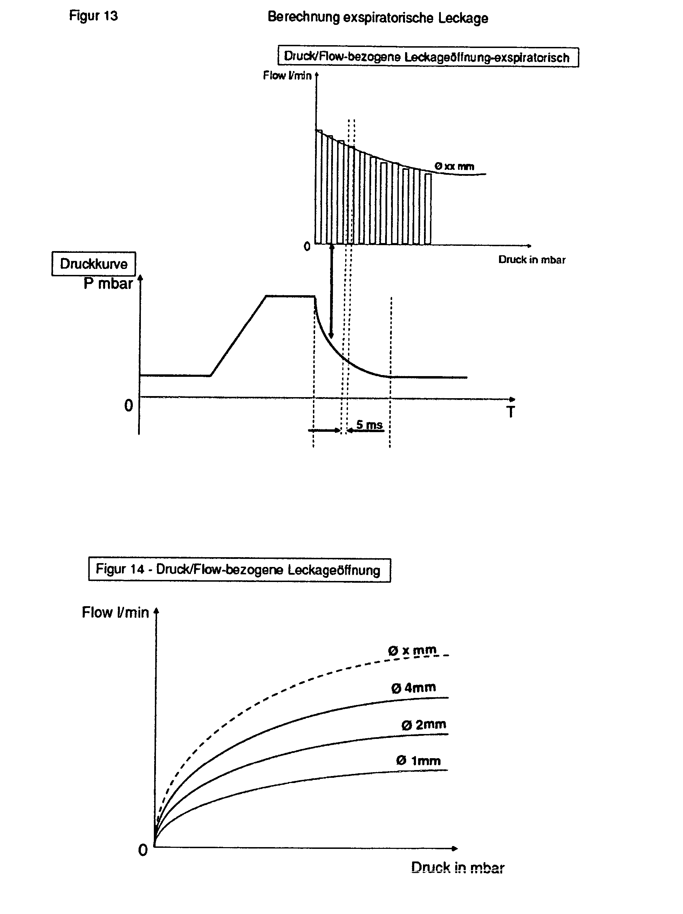

Durch den Programmteil 3 wird unter Zuhilfenahme des gemessenen Flows, der notwendig ist um einen vorgegebenen PEEP zu halten, die Leckageöffnung ermittelt (siehe Figur 3).
Ersatzweise wird bei Beatmungen ohne PEEP-Vorgabe innerhalb der Atempausen in Abständen von mindestens einer und höchstens zwei Minuten automatisch ein Prüfflow vom Beatmungsgerät initiiert, der einen PEEP von mind. 3 mbar und höchstens
6 mbar erzeugt, um eine eventuell vorhandene Leckage zu ermitteln. Im Programmteil 4 wird in Abständen von 5 ms, parallel zur inspiratorischen Druckkurve, die zur LÖ passende Druck- Flowkurve (siehe Figur 4) abgetastet. Dabei werden die Druckwerte der inspiratorischen Druckkurve als Ablesewerte auf der zur LÖ passenden Druck- Flowkurve (siehe Figur 12) für den Flow genutzt. Durch Integration über die Zeit wird das inspiratorische Leckagevolumen $V_{ti}$ Leck errechnet. Eine andere Möglichkeit der Einrichtung besteht darin, dass das zuvor genannte Verfahren beim exspiratorischen Anteil der Druck/Flowkurven (siehe Figur 13) angewandt wird.

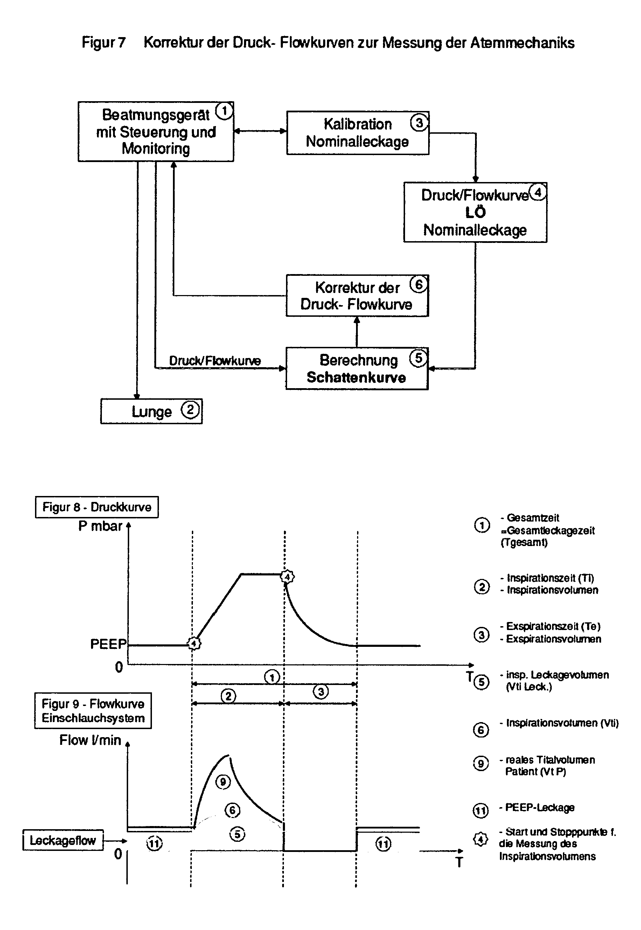

Da bei Beatmungen mit Nominalleckage eine Differenzierung zwischen einer Nominalleckage und parasitärer Leckagen notwendig ist, wird mittels Kalibrationsprogramm 7 die Nominalleckage ermittelt und die entsprechende DruckFlowkurve im Programmteil 8 zur weiteren Verwendung gespeichert. Diese DruckFlowkurve wird programmgemäß parallel zur inspiratorischen Druck- Flowkurve alle 5 ms abgetastet und es wird durch Integration über die Inspirationszeit das inspiratorische Leckagevolumen, welches durch die Nominalleckage verursacht wurde, errechnet (siehe Fig. 8). Durch automatische Differenzierung zw. der zuvor ermittelten Vti Leck, welches dem inspiratorischen Gesamtleckagevolumen entspricht und dem nunmehr bekannten Leckagevolumen, welches durch die Nominalleckage verursacht wurde, können die unterschiedlichen Anteile im Monitoring angezeigt werden.

Figur 4 und 5

In einer weiteren Ausführung der Einrichtung wird wie schon zuvor beschrieben, durch den Programmteil 3 unter Zuhilfenahme des gemessenen Flows, der notwendig ist, um einen vorgegebenen PEEP zu halten, die Leckageöffnung ermittelt (siehe Figur 3). Ersatzweise wird bei Beatmungen ohne PEEP-Vorgabe innerhalb der Atempausen in Abständen von mind. einer und höchstens zwei Minuten automatisch ein Prüfflow vom Beatmungsgerät initiiert, der einen PEEP von mind. 3 mbar und höchstens 6 mbar erzeugt, um eine eventuell vorhandene Leckage zu ermitteln.

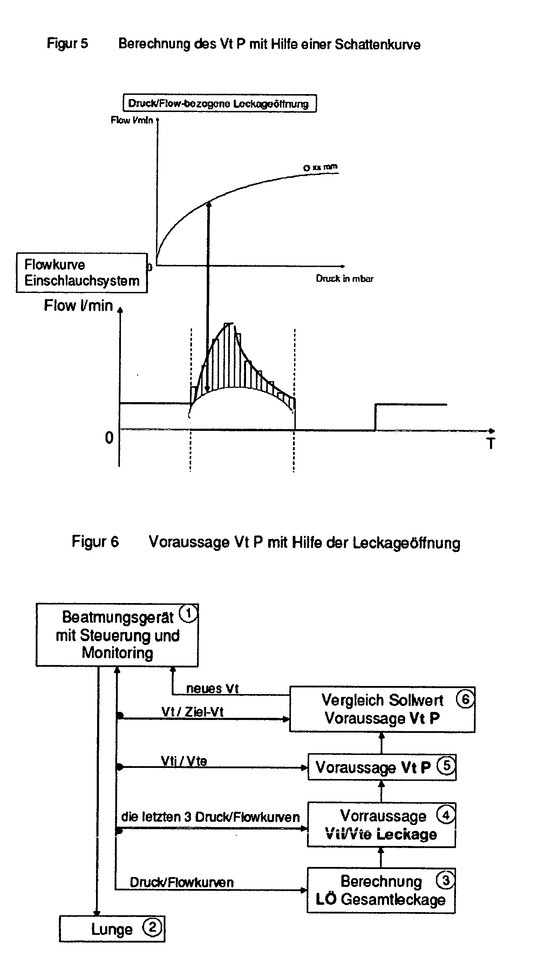

Jetzt werden die Flowwerte, die zur ermittelten LÖ gehören, parallel zur inspiratorischen Flowkurve als Schattenkurve gelegt (entspr. Anspruch 4) (siehe Figur 5). Die Nulllinie des Inspirationsflows wird danach auf die Schattenkurve der Leckage verschoben. Der am Geräteausgang gemessene Wert des inspiratorischen Volumens wird dadurch um den Wert des Leckagevolumens reduziert. Das errechnete $V_{ti}$ entspricht jetzt dem $V_t$ P.

Da bei Beatmungen mit Nominalleckage eine Differenzierung zwischen einer Nominalleckage und parasitärer Leckagen notwendig ist, wird mittels Kalibrationsprogramm 6, die Nominalleckage ermittelt und die entsprechende Druck- Flowkurve im Programmteil 7 zur weiteren Verwendung gespeichert. Diese Druck- Flowkurve wird programmgemäß genutzt, um durch Differenzbildung zw. der gemessenen LÖ der Gesamtleckage und der Nominalleckage, die LÖ, die der parasitären Leckage entspricht, zu errechnen. Die daraus resultierende korrigierte Druck- Flowkurve wird entsprechend der vorgehenden Beschreibung als Schattenkurve für die Nulllinie der inspiratorischen Druck- Flowkurve genutzt. Beispiel:

Gemessene Nominalleckage = 4 mm LÖ

Gemessene Gesamtleckage = 8 mm LÖ

Als parasitäre Leckage ergeben sich 4 mm LÖ.
Die Druck- Flowkurve einer 4 mm LÖ wird in diesem Beispiel als Nulllinie in die Inspiratorische Druck- Flowkurve gelegt.

Figur 6

Zusätzlich wird ist die genannte Einrichtung so ausgeführt, dass in bekannter Weise Druck/Flowkurven der letzten Atemzüge/Atemhübe gespeichert werden. Neu ist, dass unter Zuhilfenahme einer ermittelten LÖ und der zuvor gespeicherten Druck/ Flowkurven, eine Voraussage des nächsten Vti Leck und/oder $V_t$ P getroffen werden kann. Dadurch ist es möglich, die Korrektur für ein $V_t$ im Voraus zu berechnen und der Lunge bereits beim nächsten Atemzug ein korrigiertes Volumen zuzuführen (Volumenbeatmung) oder den Wert für ein Ziel-$V_t$ und dem damit verbundenen zulässigen Inspirationsdruck $P_{max}$ zu korrigieren (Druckbeatmung).
Dazu wird wie bereits zuvor beschrieben, im Programmteil 3 eine LÖ ermittelt.
Im Programmteil 4 werden die Mittelwerte der gespeicherten Druck/Flowkurven genutzt, um das nächste Lekkagevolumen im Programmteil 4 und daraus das nächste Vt P im Programmteil 5 vorauszuberechnen. Im Programmteil 6 wird der entsprechende Sollwert mit dem vorausberechneten Vt P verglichen und bei Abweichungen ein entsprechender Korrekturwert für den nächsten Atemzug vorgegeben.

Figur 7

Mittels Kalibration der Nominalleckage und unter Nutzung der zuvor beschriebenen Schattenkurve (siehe Anspruch 4) werden die zur Messung der dynamischen Atemmechanik einer Lunge zu analysierenden Druck/Flowkurven um die Werte der aus der Nominalleckage resultierenden Verfälschungen automatisch korrigiert.
Im Programmteil 3 wird dazu die Nominalleckage ermittelt und die entsprechende Druck- Flowkurve im Programmteil 4 zur weiteren Verwendung gespeichert.
Im Programmteil 5 wird der Flowwert in der zur LÖ passenden Druck- Flowkurve parallel zur inspiratorischen Flowkurve als Schattenkurve gelegt (siehe Figur 5). Die Nulllinie des Inspirationsflows wird danach auf die Schattenkurve der Leckage verschoben.
Die im Programmteil 6 korrigierte Druck- Flowkurve wird zur Analyse der Atemmechanik zur Verfügung gestellt.
Dadurch ist es erstmals möglich, auch im offenen Beatmungssystem die dynamischen Atemmechanik einer Lunge zu messen.

Beschreibung der Einrichtung

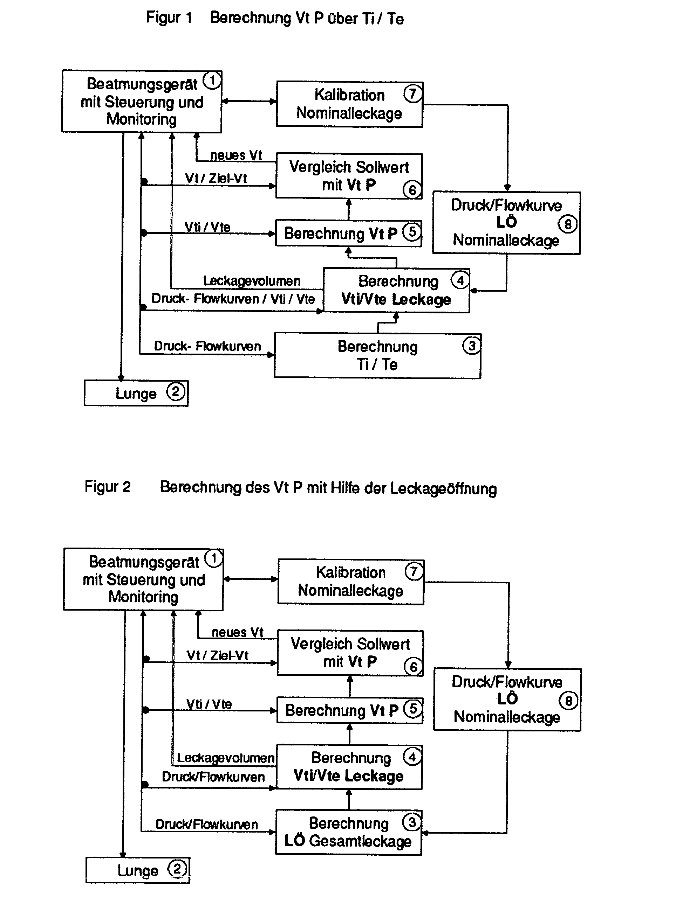

Eine erfindungsgemäße Einrichtung ist so ausgeführt, dass das ermittelte $V_t$ P im Monitoring automatisch zur Anzeige gebracht wird, was eine genauere Verlaufskontrolle der künstlichen Beatmung ermöglicht.
Die Einrichtung ist so ausgeführt, dass eine kundenspezifische Konfiguration bzgl. der Beatmung mit offenem (mit integrierter Nominalleckage) oder geschlossenem Beatmungssystem anwählbar ist. Bei Auswahl einer Beatmung mit Nominalleckage, fordert die Einrichtung automatisch zur Kalibration der Nominalleckage auf. Diese Kalibration wird in den entsprechenden Programmteilen durchgeführt (Fig. 1+2 Programmteil 7; Fig. 4 Programmteil 6; Fig. 7 Programmteil 3).
Um sicherzustellen, dass keine parasitären Leckagen das Ergebnis verfälschen, ist im Voraus durch den Anwender, das Beatmungssystem (ohne Lunge) zu installieren. Das Beatmungssystem ist zu verschließen und die automatische Kalibration zu starten. Die vorgeschlagene Einrichtung beinhaltet einen automatischen Messzyklus, der durch Erzeugung zweier Drücke nacheinander in Höhe von 10 mbar und 20 mbar mittels Flowabgabe von der integrierten Turbine oder dem pneumatischen System in das Beatmungssystem, in der Lage ist, die vorhandene Nominalleckage zu messen und einer entsprechenden Druck/Flowkurve zuzuordnen. Der Flow, der notwendig ist, um den vorgegebenen Druck zu halten, ist der Parameter zum ermitteln der Nominalleckage (Figur 3). Die beiden Ergebnisse werden gemittelt und der Mittelwert für die weiteren Berechnungen genutzt. Der Wert der Leckageöffnung, der der Nominalleckage entspricht, wird für weitere Berechnungen gespeichert.

Die Einrichtung ist so ausgeführt, dass für die Steuerung von Druckbeatmungsmodi mit integriertem Ziel-$V_t$ bei Benutzung dieser Funktion, nunmehr zur Anpassung des $P_{max}$, nicht wie bisher das evtl. fehlerbehaftete $V_{ti}$, sondern das nach Anspruch 1 ermittelte $V_t$ P genutzt wird. Die automatische Anpassung, infolgedessen sich das $V_{ti}$ beim nächsten Atemzug ändert, kann nunmehr unter objektiven Gesichtspunkten erfolgen. Die Schritte zur Anpassung des $P_{max}$ sind dabei nicht kleiner als 0,5 mbar und nicht größer als 3 mbar, je nach Höhe der Abweichung.

Weicht das ermittelte $V_t$ P trotz Korrekturregelung bei mind. 3 Beatmungszyklen hintereinander um mehr als 10 % vom Ziel-$V_t$ ab, wird ein akustischer- und optischer Alarm als Hinweis für den Nutzer/Bediener von der Einrichtung erzeugt. Eine variabel einstellbare Alarmgrenze bezüglich der Höhe der Abweichung ist vorgesehen. Die Funktion der automatischen Anpassung des $P_{max}$, auf der Grundlage des errechneten $V_t$ P ist deaktivierbar für die Fälle, in denen durch med. Probleme des beatmeten Patienten diese Funktion nicht erwünscht und/oder kontraindiziert ist.

Für die Steuerung von Volumenbeatmungsmodi ist die Einrichtung nach so ausgeführt, dass das ermittelte $V_t$ P zur automatischen Anpassung des ursprünglich vorgegebenen $V_t$ genutzt wird.

Die Schritte zur Anpassung sind dabei nicht kleiner als 5% und nicht größer als 10% des vorgegebenen $V_t$, in Abhängigkeit von der Höhe der Abweichung.

Volumenmodi, die bereits Algorithmen zur Anpassung des zulässigen Inspirationsdrucks $P_{max}$ beinhalten, w.z.B. PRVC, werden so gesteuert, dass zur Beurteilung, ob das $V_t$ durch Veränderungen der Resistance und/oder Compliance nicht erreicht wurde, oder ob diese Messergebnisse durch Leckagen verfälscht wurden, dass das berechnete $V_t$ P genutzt wird. Die automatische Anpassung eines $P_{max}$ erfolgt unter objektiven Gesichtspunkten durch Vergleich des $V_t$ und des $V_t$ P.

Die Schritte zur Anpassung sind dabei nicht kleiner als 0,5 mbar und nicht größer als 3 mbar, in Abhängigkeit von der Höhe der Abweichung.

Weicht das ermittelte $V_t$ P trotz Korrekturregelung bei mind. 3 Beatmungszyklen hintereinander um mehr als 10 % vom Ziel-$V_t$ ab, wird ein akustischer- und optischer Alarm als Hinweis für den Nutzer/Bediener von der Einrichtung erzeugt. Eine variabel einstellbare Alarmgrenze bezüglich der Höhe der Abweichung ist vorgesehen.

Die Funktion der automatischen Anpassung des $P_{max}$ auf der Grundlage des errechneten $V_t$ P ist deaktivierbar, für die Fälle, in denen durch med. Probleme des beatmeten Patienten diese Funktion nicht erwünscht und/oder kontraindiziert ist.

**Patentansprüche**

1. Verfahren zur automatischen Ermittlung eines Beatmungsvolumens, welches bei der künstlichen Beatmung mit einem Beatmungsgerät am Gasaustausch in der Lunge teilnimmt, **gekennzeichnet dadurch, dass** das Beatmungsvolumen als reales Titalvolumen unter Berücksichtigung vorhandener parasitären Leckagen und Nominalleckagen ermittelt wird.

2. Verfahren nach Anspruch 1 **dadurch gekennzeichnet, dass** unter Verwendung einer Volumenmessung für das inspiratorische- und exspiratorische Titalvolumen, einer Vermessung von Zeiten für eine inspiratorische- und exspiratorische Druckkurve unter Auslassung einer Atempause sowie einer Berechnung eines relativen Verhältnisses zwischen einer inspiratorischen und einer exspiratorischen Leckage, ein inspiratorisches- und exspiratorisches Leckagevolumen ermittelt wird, um damit ein reales Titalvolumen zu errechnen.

3. Verfahren nach Anspruch 1 **dadurch gekennzeichnet, dass** unter Verwendung eines ermittelten Durchmessers einer Leckageöffnung, ein daraus resultierendes inspiratorisches- und/oder exspiratorisches Lekkagevolumen ermittelt wird, um damit ein reales Titalvolumen zu errechnen.

4. Verfahren nach Anspruch 1 **dadurch gekennzeichnet, das** unter Verwendung eines ermittelten Durchmessers einer Leckageöffnung, eine daraus resultierende inspiratorische und/oder exspiratorische Schattenkurve erzeugt wird, welche dann wiederum zur Verschiebung einer Nullinie einer Flowkurve dient, welche zur Berechnung eines realen Titalvolumens genutzt wird.

5. Verfahren **dadurch gekennzeichnet, dass** unter Berücksichtigung von Mittelwerten bereits gemessener Druck/Flowkurven einer Beatmung und eines zeitnah ermittelten Durchmessers einer Leckageöffnung, eine Vorausberechnung eines zukünftigen Leckagevolumens zur Anpassung einer Beatmungssteuerung an die sich ändernden Bedingungen genutzt wird.

6. Verfahren einer automatischen Ermittlung einer Nominalleckage jeglicher Größe, **dadurch gekennzeichnet, dass** eine Kalibrierung einer solchen durchgeführt und ein Ergebnis für weiteren Berechnungen gespeichert wird.

7. Verfahren **dadurch gekennzeichnet, dass** bei einer Beatmung mit Nominalleckage mittels einer Kalibration einer Nominalleckage , eine daraus resultierende inspiratorische und/oder exspiratorische Schattenkurve erzeugt wird, welche dann wiederum zur Verschiebung einer Nullinie einer Flowkurve, die zur Messung von dynamischen Atemmechanik genutzt werden soll dient, um eine automatische Korrektur von auszuwertenden Ausgangsparametern für eine Messung dynamischer Atemmechanik durchzuführen.

8. Einrichtung mit einem programmierbaren Mikroprozessor zur automatischen Steuerung eines Beatmungsgerätes, **dadurch gekennzeichnet, dass** unter Verwendung eines der vorgehenden Ansprüche, die automatische Regulation eines abzugebenden Volumens oder einer zu regulierenden Beatmungsdruckbegrenzung bei künstlicher Beatmung unter Berücksichtigung vorhandener parasitärer Leckagen und unterschiedlicher Nominalleckagen durchgeführt wird, parasitäre Lekkagen, Nominalleckagen und das reale Titalvolumen angezeigt werden, sowie die auszuwertenden Ausgangsparameter für die Messung von dynamischer Atemmechanik automatisch korigiert werden.
   Es folgen 6 Seiten Zeichnungen.

Figur 1    Berechnung Vt P über Ti / Te

Figur 2    Berechnung des Vt P mit Hilfe der Leckageöffnung

Figur 3          Beispiel zur Ermittlung der Leckageöffnung

Figur 4      Berechnung des Vt P mit Hilfe einer Schattenkurve

Figur 5    Berechnung des Vt P mit Hilfe einer Schattenkurve

Figur 6    Voraussage Vt P mit Hilfe der Leckageöffnung

Figur 7    Korrektur der Druck- Flowkurven zur Messung der Atemmechaniks

Figur 8 - Druckkurve

Figur 9 - Flowkurve
Einschlauchsystem

① - Gesamtzeit
    =Gesamtleckagezeit
    (Tgesamt)

② - Inspirationszeit (TI)
    - Inspirationsvolumen

③ - Exspirationszeit (Te)
    - Exspirationsvolumen

⑤ - insp. Leckagevolumen
    (Vti Leck.)

⑥ - Inspirationsvolumen (Vti)

⑨ - reales Titalvolumen
    Patient (Vt P)

⑪ - PEEP-Leckage

④ - Start und Stopppunkte f.
    die Messung des
    Inspirationsvolumens

Figur 10 - Druckkurve

P mbar

PEEP

0

- Gesamtzeit
① =Gesamtleckagezeit
(Tgesamt)

② - Inspirationszeit (Ti)

③ - Exspirationszeit (Te)

⑤ - Inspirationsvolumen (Vti)

⑥ - inspiratorisches
Leckagevolumen (Vti Leck.)

⑦ - Exspirationsvolumen (Vte)

⑧ - Exspiratorisches
Leckagevolumen (Vte Leck.)

⑨ - reales Titalvolumen
Patient (Vt P)

⑩ Start/Endpunkte f.
Zeitmessung Ti /Te

Figur 11 - Flowkurve
Doppelschlauchsystem

Flow l/min

0

Figur 12                    Berechnung inspiratorische Leckage

Druck/Flow-bezogene Leckageöffnung

Flow l/min

Ø xx mm

0                                          Druck in mbar

Druckkurve

P mbar

0

5 ms

**Figur 13**          Berechnung exspiratorische Leckage

Druck/Flow-bezogene Leckageöffnung-exspiratorisch

Flow l/min

Ø xx mm

Druck in mbar

Druckkurve

P mbar

5 ms

T

**Figur 14 - Druck/Flow-bezogene Leckageöffnung**

Flow l/min

Ø x mm

Ø 4mm

Ø 2mm

Ø 1mm

Druck in mbar